# EUROPEAN PATENT APPLICATION

(11) **EP 3 093 664 A1**
(43) Date of publication of application: **16.11.2016**
(21) Application number: 15167673.1
(22) Date of filing: 13.05.2015
(51) Int. Cl.: G01N 33/543

(54) **A METHOD FOR ANALYSING MARKERS ON THE SURFACE OF VESICLES**

(71) Applicant: Miltenyi Biotec GmbH, 51429 Bergisch Gladbach (DE)
(72) Inventor: Bosio, Andreas, 50939 Köln (DE); Wild, Stefan, 50829 Köln (DE); Koliha, Nina, 51105 Köln (DE)
(74) Representative: Biervert, Christian

(57) **Abstract**

The present invention provides a method for analysing levels of markers of subpopulations of vesicles in a sample comprising a heterogeneous population of said vesicles, the method comprising a) contacting said sample with a set of at least two capture agents, marker of said vesicles; thereby capturing at least two subpopulations of said vesicles, b) contacting the captured at least two subpopulations of vesicles with at least two different detection agents, thereby labeling at least two markers of said at least two subpopulations of said vesicles inversely with said at least two capture agents and at least two detection agents, thereby generating an at least 2x2 matrix of signal intensities, and c) determining the levels of said at least two markers of said at least two subpopulations of said vesicles, wherein said levels of said at least two markers of subpopulations of said vesicles are determined relative to each other.

## Description

### Field of the Invention

The present invention relates to the field of cell biology, in particular to characterizing and discriminating membrane enclosed bodies like cells, intracellular compartments of cells, extracellular vesicles of cells, or enveloped viruses.

### Background of the invention

Membrane enclosed compartments are a fundamental principle in biology. First of all, cells are encompassed by the cellular membrane and the membrane takes part in various functions like matter exchange with the intercellular space, communication, cell to cell contact, cell movement etc.. Also the intracellular space is subdivided into various compartments like the nucleus, mitochondria, peroxisomes, or the endomembrane system e.g. endoplasmatic reticulum, the golgi complex, endosomes, lysosomes, and secretory vesicles. Although the membranes usually define the borders of the respective compartment, intra- and intermembrane interactions have been described allowing matter exchange between compartments. One transport mechanism are membrane enclosed vesicles resulting from engulfing or budding of intracellular membranes or the cell membrane (Holthuis JC and Ungermann C, Biol Chem. 2013 Feb;394(2):151-61).

A very recent example are mitochondria derived vesicles described to transport a mitochondrial outer membrane protein MAPL to a subpopulation of peroxisomes (Sugiura A. et al, EMBO J. 2014 Oct 1;33(19):2142-56).

Also cells release diverse types of membrane vesicles of endosomal and plasma membrane origin like exosomes, microvesicles, or viruses into the extracellular environment. Extracellular vesicles (EVs) represent an important mode of intercellular communication by serving as vehicles for transfer of membrane and cytosolic proteins, lipids, and RNA (Raposo and Stoorvogel, JBC (2013): 200:373-383).

Exosomes were found to be released by a variety of cultured cells and by many different cell types such as B lymphocytes, dendritic cells, cytotoxic T cells, platelets, mast cells, neurons, oligodendrocytes, Schwann cells, and intestinal epithelial cells.

Vesicles with hallmarks of exosomes have been isolated not only from cell culture but also from diverse body fluids, including semen, blood, urine, saliva, breast milk, amniotic fluid, ascites fluid, cerebrospinal fluid, and bile.

Currently, EVs are mostly isolated from the supernatants of cultured cells grown without serum or with serum depleted of EVs by performing differential ultracentrifugation. Next, EVs can be efficiently separated from non-membranous particles, such as protein aggregates, by using their relatively low buoyant density, and differences in floatation velocity can be used to separate differently sized classes of EVs. Additional purification can be achieved by immunoadsorption using a protein of interest, which also selects for vesicles with an exoplasmic or outward orientation.

As a consequence of their origin, exosomes from different cell types contain endosomeassociated proteins (e.g., Rab GTPase, SNAREs, Annexins, and flotillin). Membrane proteins that are known to cluster into microdomains at the plasma membrane or at endosomes often are also enriched on EVs. These include tetraspanins, a family of >30 proteins that are composed of four transmembrane domains. Tetraspanins such as CD63, CD81, CD82, CD53, and CD37 were first identified in B cell exosomes but later also in other vesicles.

It is commonly accepted that exosomes and microvesicles (MVs) represent important vehicles of intercellular communication between cells locally or at a distance. The capacity of exosomes to act as antigen-presenting vesicles, to stimulate antitumoral immune responses, or to induce tolerogenic effects has stimulated the interest of immunologists to investigate their potential use in clinics. Tumor cells as well as other cells in tumor microenvironments also secrete EVs, and there is evidence that these contribute to tumor progression by promoting angiogenesis and tumor cell migration in metastases. Tumor-derived vesicles also bear immunosuppressive molecules, which can inactivate T lymphocytes or natural killer cells, or promote the differentiation of regulatory T lymphocytes or myeloid cells to suppress immune responses. Generally speaking, there is an increasing interest in exosomes and other EVs due to their potential use in therapeutics or as biomarkers for disease (Raposo and Stoorvogel, JBC 2013: 200:373-383).

Clayton et al (2001, J Immunol Methods, 247:163-174) disclosed a method for the isolation and analysis of exosomes released by antigen-presenting cells (APC). Such exosomes originate from MHC class II peptide loading compartments and, as such, express high levels of MHC Class II. They have utilised magnetic beads, coated with monoclonal antibodies specific for HLA DP, DQ, DR for the specific isolation of exosomes from cell-free supernatants. Beads coated with exosomes were subsequently stained with conjugated antibodies, and analysed by flow cytometry. Characterisation of exosomes by this method demonstrated that exosomes derived from B-lymphocytes express abundant MHC Class I and II molecules. Other immunologically important molecules detected included the co-stimulatory molecules B7.1 (CD80) and B7.2 (CD86).

WO2010/056337 discloses a method for multiple analysis of a plurality of exosomes comprising i) applying said plurality of exosomes to a plurality of substrates, wherein each substrate is coupled to one or more capture agents, and each subset of said plurality of substrates comprises a different capture agent or combination of capture agents than another subset of said plurality of substrates; ii) capturing at least a subset of said plurality of exosomes; and iii) detecting one or more biomarkers of said captured exosomes. WO2010/056337 does not disclose a method for analysis of levels of markers of subpopulations of extracellular vesicles.

WO2012/048372 discloses an assay for detecting a disease comprising obtaining exosomes from a biological sample; providing a support having an array of a plurality of antibodies or binding fragments of antibodies; capturing exosomes on the support by binding of exosomes to one or more of the plurality of antibodies or binding fragments of antibodies; treating the captured exosomes with an agent for a selection marker indicative of a cellular origin of an exosome; detecting a subset of exosomes having the selection marker bound to the support; and identifying an array binding profile of the subset of exosomes, wherein the array binding profile is indicative of the disease. Again, WO2012/048372 does not disclose a method for analysis of levels of markers of subpopulations of extracellular vesicles.

Extracellular vesicles can be considered as a specialized biological body enclosed by a lipid bilayer like the cell membrane. Another biological body enclosed by a membrane are the biological cell itself or intracellular compartments of a cell.

Therefore, there is a need in the art for an improved method for distinguishing and characterizing different subpopulations of biological bodies enclosed by a membrane such as cells, intracellular compartments, extracellular vesicles, or enveloped viruses in a sample.

### Summary of the invention

Capturing and analyzing membrane vesicles from a sample comprising a heterogeneous composition of membrane vesicles is possible by using a set of capture agents, e.g. a set of beads (multiplex beads) coupled to binding agents. In a first step the set of beads comprising distinguishable bead types (at least two types) each type coupled with at least one binding agent for potential markers on membrane vesicles is contacted with the sample. Detection of captured membrane vesicles gives a profile of markers present in the vesicle populations (similar to Western blot or mass spectroscopy). In a second step, the vesicles on the beads (the captured, immobilized vesicles) are contacted with a detection/detecting agent (recognizing the same marker or different markers on the vesicles as the capture agents). A set of at least 2 bead types (capture agents) with different specificity and at least 2 detection agents gives a 2x2, 3x3 or higher matrix allowing detection of co-expressed markers on the membrane vesicles in a relative way to each other. For example, vesicles captured by marker A and detected by marker B means at least some of the vesicles must comprise both markers.

The matrix does not need to be balanced. Depending on the experimental design, few multiplex beads capturing the vesicles can be stained with many different detecting agents (e.g. antibodies) or several beads could also be stained by just 2 detecting agents of interest (matrices can be e.g. 2x3, 2x4, 3x4, 3x2, 4x2, 4x3). The only prerequisite is that at least two capture agents bind the at least two same markers as the at least two detection agents. Surprisingly, the matrix is not always completely symmetric if the same binding agent pair such as an antibody pair is used for vesicle capture and vesicle detection by means of swapping the capture agent pair and the detection agent pair, respectively.

Detailed experiments revealed that the amount of a given marker per vesicle and the proportion of vesicles carrying the respective marker affect the signal intensities differentially depending on whether the respective marker is used to capture the vesicle or to detect the bound vesicles. Swapping capture and detection agent can give different signal intensities and such differences can be used to deduce information on the marker distribution.

Theoretically, one marker entity is sufficient to bind a vesicle on a respective capture agent and increasing the amount of marker entities might not increase the amount of bound vesicles. In contrast, more marker entities will give brighter signals if the respective marker is used for detection. In an ideal system, signal intensity will then be directly proportional to the number of marker entities per vesicle.

In the following, the principle of the method of the present invention is exemplified by analysing exosomes as a type of vesicles. Exosomes comprising two markers A and B with a 100 marker entities A and 1 marker entity B on each vesicle will ideally give comparable signal intensities if the detection agent specific for marker A is used. As all vesicles comprise at least one entity of each marker, exosomes will be captured to the same extent by capture agent A and B. The same number of captured exosomes and all vesicles comprise 100 marker entities A will result in comparable, strong signal intensities.

If swapping capture and detection agent having the same specificity for the respective marker, again exosomes will be captured to the same extent by capture agent A and B. However, now detecting marker B all vesicles comprise only one marker entity B giving comparable, but weak signal intensities.

**Table 1**

| | A staining | B staining |
|---|---|---|
| A bead | 100 | 1 |
| B bead | 100 | 1 |

Table 1 shows a theoretical example of a 2x2 matrix for marker A and B with marker B being less pronounced per vesicle. The numbers haven been chosen assuming the signal intensity reflecting the numbers of the respective marker per vesicle.

In general, if less entities of a marker are present on the vesicles, signal intensities will drop by swapping the respective capture agent and use this marker for detection. The number of captured exosomes is not affected as the extent of bound vesicles will be the same for both capture agents.

On the other hand, the proportion of vesicle comprising a given marker will also affect the signal intensities if capture and detection agent are swapped.

A sample comprising two populations of vesicles: A first population of 50% of all vesicles with vesicles carrying 100 entities of marker A and 100 entities of marker B on each vesicle and a second population with the remaining 50% of all vesicles carrying 100 entities of marker A per vesicle but no marker B.

Using capture agents for marker A will capture double the amount of vesicles as compared to capture agents for marker B. Therefore, detection using detection agents specific for marker A with 100 entities on all exosomes will give double the signal on capture agents for marker A as compared to the capture agents for marker B.

If marker B is used for detection, only 50% of the vesicles bound to capture agents specific for marker A will be detected as the remaining vesicles are devoid of marker B and will give no signal. A comparable signal can be expected for capture agent B. In this case, only 50% of the vesicles will be bound, but all these vesicles carry marker B and will be detected.

**Table 2**

| | A staining | B staining |
|---|---|---|
| A bead | 100 | 50 |
| B bead | 50 | 50 |

Table 2 shows a theoretical example of a 2x2 matrix for marker A and B with marker B being less pronounced per vesicle. The numbers haven been chosen assuming the signal intensity reflecting the numbers of the respective marker per vesicle.

By swapping capture agent and detection agent, signal intensities drop if only a subpopulation comprises one of the markers and the marker present only on the subpopulation is used for capture but not for detection. If the subpopulation marker is used for detection the signal intensities are comparable for both capture agents. Capture agent specific for marker A present on all vesicles captures a representative portion of the exosome sample comprising 50% of vesicles negative for marker B. Detection by the agent specific for marker B will consequently detect only half of the bound exosomes. Capture agent specific for marker B present on only 50% of all vesicles captures just the 50% of vesicles positive for marker B. Detection by the agent specific for marker A will detect all of the bound exosomes, again giving half of the signal gained for all exosomes bound and detected by marker A.

In general, the proportion of vesicles carrying a marker ideally will be preserved on the capture agents in case any other marker is used to capture the vesicles. However, when the marker, which is present only on a vesicle subpopulation, will be used for detection, only this subpopulation can be stained giving a lower signal intensity. Swapping the binding agents specific for two markers between capture agent and detection agent and using the marker present only on a vesicle subpopulation, only this subpopulation will be bound and the signal will be lower independently of the used detection marker.

The effect is also observed if the matrix is not balanced, it is sufficient that at least two capture agents which recognize different markers on the vesicles and at least two detection agents which recognize different markers on the vesicles are used, wherein at least two of said capture agents and two of said detection agents recognize at least two of the same markers, respectively.

The invention allows to compare the abundance of at least two markers per vesicle and to determine the proportion of at least two subpopulations defined by at least one marker and enables to discriminate less markers per vesicles from a subpopulation e.g. less vesicles comprising the marker if at least two capture agents recognizing different markers on the vesicles recognize the same markers as at least two detecting agent.

Using such a matrix we can record more detailed marker profiles of different vesicles, e.g. extracellular vesicle populations (e.g. healthy vs. disease, extracellular vesicles of different cell types, or extracellular vesicles +/- stimulus).

Exemplarily, we have compared one subset of beads with sets of 8 and sets of 39 beads giving comparable signal intensities independently of the number of different beads. We also tested a mixture of 39 subsets of beads and used double or four times the amount of total beads again giving comparable signal intensities independently of the number of beads. We concluded that 4x39 = 156 different subsets of beads still would give results independently from the higher number of different subsets of beads used. Larger bead sets are known in the art like HumanHT-12 v4 Expression BeadChip (Illumina) comprising 47,231 different bead sets. The method of the present invention is not limited to beads, any surface suitable for coupling the binding agents to the surfaces of the capture agents and applicable in the method can be used instead of beads.

The method of the present invention is not limited to analyse subpopulations of extracellular vesicles, any vesicle which has markers on the vesicle membrane may be used for analysis. The vesicles may be cells, intracellular compartments, extracellular vesicles of cells, or enveloped viruses.

### Brief description of the Drawings

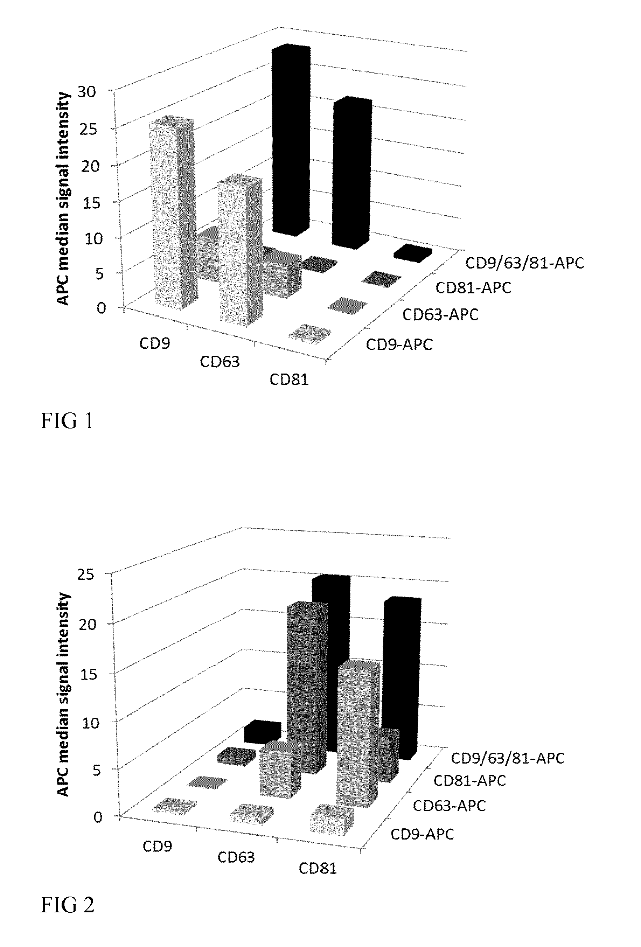

FIG 1: Background corrected APC median signal intensities of platelet vesicles bound to antiCD9-, antiCD63- and antiCD81-beads after staining with a cocktail of antiCD9-APC (Miltenyi Biotec, Germany), antiCD63-APC (Miltenyi Biotec, Germany) and antiCD81-APC (BD) or with each detection antibody alone.
FIG 2: Background corrected APC median signal intensities of NK cell exosomes bound to antiCD9-, antiCD63- and antiCD81-beads after staining with a cocktail of antiCD9-APC (Miltenyi Biotec, Germany), antiCD63-APC (Miltenyi Biotec, Germany) and antiCD81-APC (BD) or with each detection antibody alone.

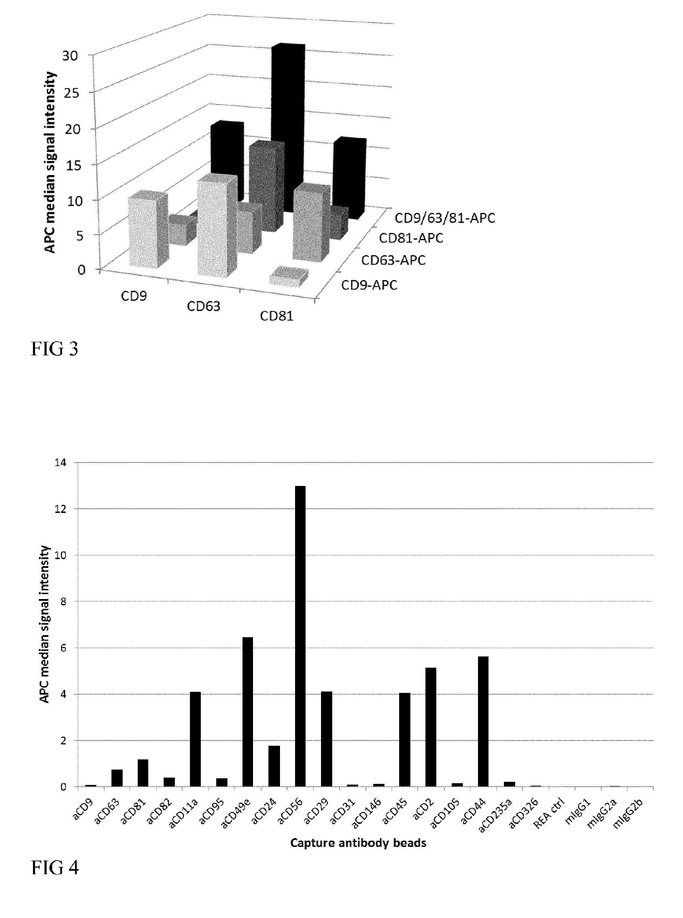

FIG 3: Background corrected APC median signal intensities of a 1:1 mixture of platelet vesicles and NK cell exosomes bound to antiCD9-, antiCD63- and antiCD81-beads after staining with a cocktail of antiCD9-APC (Miltenyi Biotec, Germany), antiCD63-APC (Miltenyi Biotec, Germany) and antiCD81-APC (BD) or with each detection antibody alone.
FIG 4: Background corrected APC median signal intensities of 5x10⁶ NK cells bound to different capture beads after staining with antiCD56-APC (Miltenyi Biotec, Germany), a = anti, ctrl = control

### Detailed description of the Invention

We aimed to set up a platform allowing to investigate several surface markers on vesicles like exosomes. To this aim, we used polystyrene particles stained with different colours to distinguish each particle population e.g. using a flow cytometer. Each particle can then be coupled to a binding agent giving multiple particle types , i.e. set a of capture agents is generated. When incubating these particles with vesicles, e.g. with extracellular vesicles of a cell each particle (each capture agent) will bind vesicles carrying the respective marker. In parallel or after washing, the bound vesicles can be stained by a detection agent, e.g. a binding agent coupled to a fluorescent dye. For analysis, each particle (capture agent) can be identified by its staining colour. In addition, the fluorescent dye of the detection agent allows detection of bound vesicles. The analysis can be performed using a flow cytometer, e.g. MACSquant® Analyzer (Miltenyi Biotec GmbH). Other technologies like multicolour fluorescent imaging could also be used. Using e.g. multiplex beads, one can compare the presence of surface markers of e.g. vesicles originating from different cell types or from cells under various conditions. Besides the detection of markers, it is also possible to compare the relative amounts of markers per vesicle for different vesicle samples. In addition, it is possible to determine whether two markers are present on the same vesicles. Only if the marker used to capture the vesicle and the other marker used for staining are present on the same vesicles a signal will be detectable. As this is a valuable information, we used several staining antibodies (detection agents) in combination with multiple capture beads (capture agents). Thereby, we could define several pairs of markers present on the exosomes. To our surprise, we discovered the same pair of antibodies in some cases giving differing signal intensities if capture and detection antibody (binding agent) was swapped.

E.g. using the same antibody pair for vesicle capture and vesicle detection can give rise to a bright signal on beads coupled with antibody A (capture agent A) stained with antibody B (detection agent B) and lower signals for antibody B beads (capture agent B) stained with antibody A (detection agent A).

In contrast to most of the common multiplex analytes e.g. small molecules, mRNAs, miRNAs, cytokines, etc. being single molecules, vesicles comprise a plurality of same or different molecules. Therefore, an ideal assay would not only measure vesicles as a single unit, but give also information on the vesicle composition. Our observation of asymmetric signal intensities is usually not observed and ideally should not occur for multiplex assay measuring single molecules. In the case of a cytokine bead assay, a given cytokine will be captured by the respective antibody and stained using a complementary antibody binding on a different epitope but on the same cytokine. For each cytokine, such a pair of antibodies is used for capture and detection, respectively. If only a single kind of cytokine is in the sample, there will be a signal only on the respective bead.

For nucleic acid detection, e.g. miRNA or mRNA microarrays, specificity is not always highly discriminatory and some crosstalk between closely related sequences can be observed. For example some members of the let-7 miRNA family differ only by one nucleotide. If one miRNA will be hybridized on a miRNA array comprising complementary sequences for the different family members, a strong signal is observed on the respective spot, but some weak signals can be observed as well for the closely related family members. Typically, the RNA labeling is not sequence specific but dyes are incorporated by a polymerase, ligation or chemical reactions with the nucleic acids and all nucleic acids will be labeled. In such a setting an asymmetry is a priory not possible as only a single detection reagent is used.

So to our knowledge multiplex assay as they have been used prior to our invention usually do not provide asymmetric data.

Our observation and following experiments showed that asymmetry of the matrix e.g. changes in signal intensities by swapping capture and detection agents provide additional information on the distribution of the markers on the vesicles.

For example a binding agent, such as an antibody A or a fragment thereof recognizes a general vesicle marker A. Capture agents, e.g. beads coupled with this antibody or fragment thereof can therefore capture all vesicles (assumed for following cases 1, 2 and 3). Using the same antibody or fragment thereof for detection, all vesicles will be detected. In order to compare signal intensities of different capture agents or detection agents within an experiment or to simplify the comparison of samples, the highest signal can be set to 100% and the relative signal intensity of the remaining capture agents or detection agents can be calculated accordingly. To improve capture and/or detection of all vesicles, it is also possible to use a mixture of capture and/or detection agents and set the value for the respective signal intensity to 100%.

We set a few assumption to simplify the analysis.
- The captured vesicles hardly affect the vesicle concentration as only a small proportion of vesicles will be captured.
- Capture agents, detection agent and vesicle amount or concentration are adjusted to enable signal detection and to avoid saturation of a capture agent or depletion of the detection agent.
- The amount of capture agents is set to enable reliable signal detection
- The assay conditions are set to keep the signal gained by the detection agent proportional to the number of bound detection agents.
- The affinity of the capture agents to each respective marker is comparable.
- The affinity of the detection agents to each respective marker is comparable.
- Capture agent and detection agent should not interfere with each other

Whereas these assumptions might not be always perfectly fulfilled, the conclusion still should hold true. However, experimental conditions clearly violating the assumptions will strongly bias the results. Therefore, experiments need to be carefully designed and controlled.
- To minimize interference of the different capture agents, vesicles should be used in excess over the capture agents. Ideally, only a small portion of vesicles will be bound hardly affecting the vesicle concentration in solution. The number of marker entities present on the vesicles should be at least as high as the number of binding agents, a surplus of marker entities of at least 10-fold would be preferred, more preferred at least 100-fold, further more preferred at least 1000-fold, and most preferred at least 10000-fold.
- For each capture agent, at least one bead needs to be analyzed. To avoid gaps due to missing beads, it is preferred to use at least 5 beads per capture agent, more preferred 10 beads per capture agent, further more preferred 100 beads per capture agent, and most preferred 1000 beads per capture agent.
- A stable link is required between the binding agent of the detection agent and the signalling label, e.g. a fluorophore linked to the binding agent e.g. by a covalent bond, a coordinative bond, streptavidin biotin interaction, antibody antigen interaction or similar links. It is also possible to use signal amplification means to improve sensitivity.
- A stable link is required between the binding agent of the capture agent and the surface, e.g. a binding agent linked to a bead e.g. by a covalent bond, a coordinative bond, streptavidin biotin interaction, antibody antigen interaction or similar links. Surprisingly, it was found that the use of covalently coupled binding agents of the capture agents, e.g. beads, works better within the present method as the use of indirectly coupled systems such as streptavidin/biotin. Therefore, preferentially, the binding agents are covalently coupled the surface.
- The capture agents should be selected to have comparable affinities. If this cannot be fulfilled, lower signal intensities can be assigned to lower affinities. It is possible to correct for differing affinities e.g. by using standard curves of the markers or by theoretical calculations. The affinity of the capture agents should be at least within a range of +/- 100-fold as compared to the mean affinity. A range of the affinity of the capture agents compared to the mean affinity of capture agents of +/- 50-fold would be preferred, more preferred of +/- 10-fold,further more preferred of +/- 5-fold, and most preferred of +/- 2-fold.
- The detection agents should be selected to have comparable affinities. If this cannot be fulfilled, lower signal intensities can be assigned to lower affinities. It is possible to correct for differing affinities e.g. by using standard curves of the markers or by theoretical calculations. The affinity of the detection agents should be at least within a range of +/- 100-fold as compared to the mean affinity. A range of the affinity of the detection agents compared to the mean affinity of detection agents of +/- 50-fold would be preferred, more preferred of +/- 10-fold, further more preferred of +/- 5-fold, and most preferred of +/- 2-fold.
- To minimize blocking effects or potential interference of the capture and/or detection agents recognizing the same marker, capture and detection agents recognizing different regions or epitopes of the same marker are preferred.

### Case1: A first marker is less abundant per vesicle than a second marker

Antibody B or a fragment thereof recognizes a marker B present on all vesicles, but less abundant as compared to marker A. Although the number of vesicles bound to the capture agents, e.g. beads, will be comparable, the combination of A beads (capture agent recognizing marker A) with B antibody staining (detection agent recognizing marker B) will give a lower signal, as less markers can be stained. The other way round, B beads (capture agent recognizing marker B) will also bind all vesicles but all bound vesicles will be brightly stained by antibody A (detection agent recognizing marker A). So the signal will be comparable for both kinds of beads using antibody A for staining, but staining with antibody B gives rise to lower signals due to the lower number of B markers on the vesicle.

**Table 3**

| | A staining | B staining |
|---|---|---|
| A bead | 100 | 1 |
| B bead | 100 | 1 |

Table 3 shows a theoretical example of a 2x2 matrix for marker A and B with marker B being less pronounced per vesicle. The numbers haven been chosen assuming the signal intensity reflecting the numbers of the respective marker per vesicle.

### Case2: A marker is present only on a vesicle subpopulation

Antibody B or a fragment thereof recognizes a marker B present only on a subpopulation of the vesicles, but is abundant on the positive vesicles. The combination of A beads with B antibody detection will give a bright signal, yet lower as the AA combination as only a subset of bound vesicles is detected. The other way round, B beads will bind only a fraction of all vesicles (most of the vesicles are B negative) but all bound extracellular vesicles will be intensely detected by antibody A. So the signal will be comparable for both combinations AB or BA, but lower as compared to AA due to only a subpopulation of vesicles comprising marker B.

**Table 4**

| | A staining | B staining |
|---|---|---|
| A bead | 100 | 50 |
| B bead | 50 | 50 |

Table 4 shows a theoretical example of a 2x2 matrix for marker A and B with marker B being present only on a subpopulation of vesicles. The numbers haven been chosen assuming the signal intensity reflecting the numbers of the respective marker per vesicle.

### Case3: A first marker is present only on a vesicle subpopulation and is less abundant per vesicle than a second marker

Antibody B or a fragment thereof recognizes a marker B present only on a subpopulation of the vesicles, and in addition is scarce on the positive vesicles. The combination of A beads with B antibody detection will give a low signal. The other way round, B beads will bind only a fraction of all vesicles (most of the vesicles are B negative) and although all bound vesicles will be stained by antibody A, the signal will also be lower. The effect is strongest using marker B for capture and detection as here only a subpopulation will be bound and the respective marker is also less pronounced.. So the signal will be comparable for both kinds of staining antibodies if the same marker will not also be used to capture the vesicles. Binding and detection with antibody B gives rise to the lowest signals due to the lower amount of B positive vesicles and the lower amount of the marker B per vesicle.

**Table 5**

| | A staining | B staining |
|---|---|---|
| A bead | 100 | 50 |
| B bead | 50 | 10 |

Table 5 shows a theoretical example of a 2x2 matrix for marker A and B with marker B being less pronounced per vesicle and marker B being present only on a subpopulation of vesicles. The numbers haven been chosen assuming the signal intensity reflecting the numbers of the respective marker per vesicle.

It is also possible to combine capture agents or detection agents recognizing the same or different markers in order to increase signal intensity or to allow analyzing subpopulations of vesicles defined by a combination of markers. Such subpopulations could be for example using antiCD2, antiCD3, antiCD4, and antiCD8 antibodies for T cells or antiCD19 and antiCD20 for B cells.

In our experiment, we came across two striking examples. Exosomes derived from natural killer (NK) cell supernatant appeared to hardly give any signal using antiCD9 antibodies for capture or for detection. For exosomes derived from thrombocyte supernatant we observed no signal using antiCD81 antibodies for capture or for detection. We thought to use these exosomes to set up a test system simulating a mixture of two exosome subpopulations. We mixed the same amount (based on protein content) of NK cell derived and thrombocyte derived exosomes and incubated this exosome mixture with multiplex beads as capture agents each coupled to antiCD9, antiCD63, antiCD81, and other antibodies, respectively. We then stained the exosome mixture with APC-coupled antiCD9, antiCD63, antiCD81 antibodies as detection agents, respectively, or a cocktail of these three antibodies and analyzed the samples on a MACSQuant® Analyzer (Miltenyi Biotec GmbH). Detection using the antiCD9, antiCD63, and antiCD81APC antibody cocktail as detection agent as well as antiCD63 antibody APC as detection agent showed exosomes to be bound to the antiCD9, antiCD63, and antiCD81antibody coupled beads. If instead antiCD9 antibody APC was used for detection, CD9 and CD63 antibody coupled beads gave signals, but hardly any exosomes could be detected on the antiCD81 antibody bead. On the other hand, if antiCD81 antibody APC was used for detection, antiCD9 and antiCD63 antibody coupled beads gave signals, but no exosomes could be detected on the antiCD9 antibody bead.

**Table 6**

| | CD9-, CD63-, and CD81-APC | CD9 APC | CD81 APC | CD63 APC |
|---|---|---|---|---|
| CD9 bead | 13.0 LU | 9.8 LU | 0.7 LU | 3.1 LU |
| CD81 bead | 12.1 LU | 1.1 LU | 3.8 LU | 10.1 LU |
| CD63 bead | 26.5 LU | 13.3 LU | 13.0 LU | 6.2 LU |

Table 6 shows an example of a 3x4 matrix gained using a mixture of CD9 antibody beads, CD63 antibody beads, and CD81 antibody beads incubated with exosomes derived from natural killer (NK) cell supernatant. The mean signal intensity gained for each subset of capture agents using a cocktail of antiCD9, antiCD63, and antiCD81APC antibodies, or antiCD9, antiCD63, or antiCD81APC antibody alone for detection is given in arbitrary light units (LU) as measured on the MACSQuant® Analyzer. The signals are corrected for the control detection reaction using buffer only instead of exosomes. The mean signal intensity gained for each bead population in the buffer control reaction were subtracted from the mean signal detected for the exosome samples for the respective bead population.

This example nicely demonstrates results gained by single detection reactions and the potential of additional information that can be drawn from even a small 3x4 matrix. Single detections using the antiCD9 APC, antiCD63 APC, and antiCD81APC antibody cocktail or the antiCD63 antibody APC alone give positive signals for all the three beads coupled to antiCD9, antiCD63, and antiCD81 respectively suggesting the used exosome sample comprises exosomes carrying these three markers. Single detection using antiCD9 antibody APC would suggest the exosomes being negative for antiCD81 (or antiCD9). Complementary, antiCD81 antibody APC staining would suggest the exosomes being negative for antiCD9 (or antiCD81). Only the combined analysis of at least the 2x2 matrix using antiCD9 and antiCD81 for capture and detection reveals the information of at least two subpopulations of exosomes being either antiCD9 positive (in this example the exosomes derived from thrombocytes supernatant) or antiCD81 positive (in this example the exosomes derived from NK cells supernatant) but hardly any exosomes comprising both markers.

Interestingly, setting the highest signal (antiCD63 antibody beads as capture agents stained with the APC detection cocktail of antiCD9, antiCD81 and antiCD63) to 100%, one could calculate based on the corresponding signals for the antiCD9 or antiCD81 beads 49% of the exosomes also express antiCD9 and 46% of the exosomes also express antiCD81 nicely reflecting the 1:1 mixture of antiCD9 positive thrombocyte exosomes and antiCD81 positive NK cell exosomes.

In this case, the 2x2 matrix using antiCD9 and antiCD81 is nearly symmetric as both exosome populations are nearly completely devoid of one of the matrix markers. Using this artificial test system, we could show that it is indeed possible to discriminate vesicle subpopulations using our approach.

In another example, thrombocyte exosomes were incubated with a mixture of multiplex beads (capture agents) each bead coupled to antiCD9, antiCD63, antiCD81. We then stained the mixture with APC-coupled antiCD9, antiCD63, antiCD81 antibodies (detection agents), or a mixture of these three antibodies and analysed the samples on a MACSQuant Flowcytometer.

**Table 7**

| | CD9-, CD63-, CD81- APC | CD9 APC | CD81 APC | CD63 APC |
|---|---|---|---|---|
| CD9 bead | 28.4 LU | 25.4 LU | 0.2 LU | 6.4 LU |
| CD81 bead | 0.7 LU | 0.3 LU | 0.2 LU | -0.2 LU |
| CD63 bead | 21.9 LU | 19.1 LU | 0.3 LU | 4.8 LU |

Table 7 shows an example of a 3x4 matrix gained using a mixture of CD9 antibody beads, CD63 antibody beads, and CD81 antibody beads incubated with exosomes derived from thrombocyte cell supernatant. The mean signal intensity gained for subset of capture agents using a cocktail of antiCD9, antiCD63, and antiCD81APC antibodies, or antiCD9, antiCD63, or antiCD81APC antibody alone for detection is given in arbitrary light units (LU) as measured on the MACSQuant Flow Cytometer. The signals are corrected for the control detection reaction using buffer only instead of exosomes. The mean signal intensity gained for each subset of capture agents in the buffer control reaction were subtracted from the mean signal detected for the exosome samples for the respective bead population. Negative signal intensities mean the signal gained for the exosome samples has been lower as compared to the buffer control reaction.

In this case, the 2x2 matrix using antiCD9 and antiCD63 gives differing signal intensities for antiCD9 antibody beads (capture agent) stained with antiCD63 antibody APC (6.4 arbitrary light units as measured on the MACSQuant® Analyzer (LU)) and antiCD63 antibody beads (capture agent) stained with antiCD9 antibody APC (19.1 LU). This indicates antiCD63 being either less pronounced within each exosome and/or only an exosome subpopulation comprises CD63. The detection agent cocktail can be used to estimate the proportion of exosomes carrying the respective marker. As the signal for the antiCD63 antibody beads (capture agent) (21.9 LU) is lower than the signal for the antiCD9 antibody beads (capture agent) (28.4 LU), we deduce CD63 being detected only on a subpopulation of the thrombocyte derived exosomes. Setting the highest signal (antiCD9 antibody beads (capture agent) stained with the APC detection agent cocktail) to 100%, one could calculate the proportion of CD63 comprising exosomes as the proportion of the mean CD63 antibody APC (detection agent) signal intensity vs. the mean CD9 antibody APC (detection agent) signal e.g. 77% of the exosomes also express CD63. Taking the antiCD63 antibody beads (capture agent), antiCD9 antibody APC (detection agent) staining gives a brighter signal (19.1 LU) as compared to staining with antiCD63 antibody APC (detection agent) (4.8 LU) indicating also CD63 being less pronounced per exosomes as compared to CD9.

Using the same antibody for capturing and staining can lead to epitope blocking and could be an alternative explanation for the lower antiCD63 APC (detection agent) signal on the antiCD63 antibody beads (capture agent). To exclude this possibility, two different antiCD63 antibodies (binding agents) recognizing different epitopes could be used.

In this case, the same trend of antiCD63 antibody APC (detection agent) giving lower signals as compared to antiCD9 antibody APC (detection agent) can be observed for the antiCD9 antibody beads (capture agents) as well corroborating CD63 being less pronounced per exosomes as compared to CD9.

In another example, we analyzed exosomes derived from monocyte cell culture supernatant. Using the detection agent cocktail to estimate the proportion of exosomes carrying the respective markers the highest signal (antiCD63 antibody beads (capture agents) stained with the APC detection agent cocktail) is set to 100%. The relative proportion of CD9 and CD81 subpopulations can then be calculated to 69% respectively 65% of the CD63 positive exosomes. Taking the antiCD63 antibody beads (capture agents), antiCD9 antibody APC (detection agents) staining gives a brighter signal (17.9 LU) as compared to staining with antiCD63 antibody APC (detection agents) (14.9 LU) indicating also CD63 being less pronounced per exosomes as compared to CD9.

**Table 8**

| | CD9-, CD63, CD81- APC | CD9 APC | CD81 APC | CD63 APC |
|---|---|---|---|---|
| CD9 bead | 24.7 LU | 13.3 LU | 6.1 LU | 6.0 LU |
| CD81 bead | 23.3 LU | 18.1 LU | 1.1 LU | 7.9 LU |
| CD63 bead | 35.9 LU | 17.9 LU | 6.7 LU | 14.9 LU |

Table 8 shows an example of a 3x4 matrix gained using a mixture of CD9 antibody beads, CD63 antibody beads, and CD81 antibody beads incubated with exosomes derived from monocytes supernatant. The mean signal intensity gained for each subset of capture agents using a cocktail of antiCD9, antiCD63, and antiCD81APC antibodies, or antiCD9, antiCD63, or antiCD81APC antibody alone for detection is given in arbitrary light units (LU) as measured on the MACSQuant Flow Cytometer. The signals are corrected for the control detection reaction using buffer only instead of exosomes. The mean signal intensity gained for each subset of capture agents in the buffer control reaction were subtracted from the mean signal detected for the exosome samples for the respective bead population.

Further analysis of the marker distribution with the exosome subpopulations reveals less CD81 as compared to CD9 independently of the respective capture beads. In this case, the lower signal for antiCD81 antibody APC (detection agent) on antiCD81 antibody beads (capture agent) is likely due to blocking effects using the same antibody (binding agent) for capture and detection. Interestingly, for antiCD63 antibody APC (detection agent) and antiCD63 antibody beads (capture agents), the signal is still higher as compared to antiCD9 or antiCD81 antibody beads (capture agents) stained with antiCD63 antibody APC (detection agent) indicating the higher proportion of CD63 positive exosomes and/or more CD63 per exosome overcompensating a potential blocking effect. The antiCD63 antibody APC (detection agent) staining allows even to conclude that less CD63 is present per exosome for the CD9 and/or CD81 positive exosomes as the signal intensities is much lower for these beads as compared to the antiCD63 antibody bead (capture agents).

As these matrix data become quite complex, the data can be simplified and summarized e.g.
- CD63 beads (capture agents): 100% positive; CD9 high, CD81 medium
- CD9 beads (capture agents): 69% positive; CD81 medium, CD63 medium
- CD81 beads (capture agents): 65% positive; CD9 high, CD63 medium

We define signals of at least 50% of the maximal signal intensity as "high", between 10% and 50% as medium and below 10% as low.

For all the above examples we used small extracellular vesicles or exosomes, However, the invention is not limited to small vesicles. One could consider a cell as a very large vesicle and therefore, we used NK cells as an example of large vesicles. Indeed, using 39-plex beads (set of 39 subsets of beads) and antiCD56 antibody APC as a marker to stain NK cells, signals for CD45, CD2, CD11a, CD56 etc. known to be expressed on NK cells could be obtained indicating the assay to be not limited to the size of small vesicles (see Example 5). Other options for vesicles are e.g. intracellular compartments of a cell or enveloped viruses.

In an aspect the present invention provides a method for analysis of levels of markers of subpopulations of vesicles in a sample comprising a heterogeneous population of said vesicles, the method comprising
a) Contacting said sample with a set of capture agents, wherein each distinguishable subset of said set of at least two capture agents comprises a different binding agent or combination of binding agents, and wherein each different binding agent recognizes a different marker of said vesicles; thereby capturing at least two subpopulations of said vesicles of said sample
b) Contacting the captured at least two subpopulations of vesicles with at least two different detection agents, wherein each different detection agent comprises a different binding agent, and wherein each different binding agent recognizes a different marker of said vesicles, and wherein at least two of said different binding agents of said at least two detection agents recognize the same at least two markers of said vesicles as two of said different binding agents of said at least two capture agents, and wherein each binding agent of said detection agents is labeled with different or the same signaling labels; thereby labeling at least two markers of said at least two subpopulations of said vesicles inversely with said at least two capture agents and at least two detection agents, thereby generating an at least 2x2 matrix of signal intensities
c) Determining the levels of said at least two markers of said at least two subpopulations of said vesicles, wherein said levels of said at least two markers of subpopulations of said vesicles are determined relative to each other

Said signaling labels may be e.g. fluorophores, quantum dots, radioactive signals or enzymes generating detectable signals. Said signaling labels may be directly or indirectly coupled to said detection agents.

An example for an enzyme generating detectable signals may be enzyme horseradish peroxidase (HRP). HRP catalyzes the conversion of chromogenic substrates (e.g., TMB, DAB, ABTS) into colored products, and produces light when acting on chemiluminescent substrates (e.g. ECL).

The levels of the markers may be determined by using a device capable of measurement of signal intensities such as fluorescence detected by microarray scanners, microscopy, nanoscopy, mass spectroscopy, fluorescent activated cell sorter, flow cytometers, or devices capable to determine products of enzymatic reactions such as dyes or ions.

The highest signal obtained by measurement of the fluorescence of one or a combination of the detection agent(s) may be set to 100% and all other relative signal intensities may be calculated accordingly therefrom.

Said vesicles may be cells or extracellular vesicles, intracellular compartments of a cell or enveloped viruses.

Said set of capture agents may be a set of beads. The preferred size of the beads is the range detectable in a flow cytometer typically 1 µm up to 16 µm

Said distinguishable subset of said set of beads may be differently stained with dyes and /or may be different in size.

Said binding agents may be antibodies or fragments thereof.

Said binding agents of the capture agents may be covalently coupled to said beads.

Said binding agents of the detection agents may be non-covalently coupled to said beads. Said generated matrix may be a balanced matrix.

Said generated matrix may be a non-balanced matrix.

Each subset of said set of capture agents may comprise more than one molecule of said binding agents, preferentially more than 10 molecules of said binding agents, more preferentially more than 100 molecules of said binding agents.

A single capture agent ideally should be able bind to more than one marker entity. Typically, several binding agents will be coupled to the same surface to enable capture of multiple vesicles

A binding agents itself can also be able to bind more than one marker epitopes, e.g. one antibody can bind two marker epitopes. A binding agent could also be four biotinylated antibodies linked together by streptavidin resulting in a binding capacity of 4x2 epitopes and even larger complexes could be used.

### Embodiments

Depending on the surface, capture and detection agents, suitable means to discriminate the capture agents and to detect the staining agents will be used. Many devices well known in the art like microarray scanners, microscopy, nanoscopy, mass spectroscopy, fluorescent activated cell sorter, flow cytometers, microelectronic devices, etc. or combinations thereof can be used. It is also possible to use different means in parallel or one after the other to improve the discriminatory power and increase the number of potential markers to be analysed. Magnetic and non-magnetic beads of different size and colour could be mixed and incubated together with the vesicle sample and detecting agent(s). By magnetic sorting, two samples will be generated and each of the samples can then be analyzed on a flow cytometer. Within the flow cytometer, beads of different sizes can be discriminated according to their light scattering properties and in addition each kind of bead can be assigned by the respective fluorescent signal(s). Finally, the staining signal (detection signal) for each bead can be recorded. Many other combinations of bead properties and analysis means can easily be used by a person skilled in the art.

In one embodiment of the present invention a sample comprising subpopulations of vesicles is contacted with a set of beads (a bead array, multiplex beads) such as polystyrene particles of different size and/or stained with different dyes and/or intensities of dyes. Each bead (a subset) is coupled to a binding agent such as an antigen binding molecule, e.g. an antibody or fragment thereof, which is specific for a potential marker on a vesicle. The set of beads may comprise two beads to as many beads allowing independent detection of each bead.

The set of beads may comprise up to 100, 500, 1000, 5000, 10000, 50000 or 100000 distinguishable beads.

Each bead is decorated with different binding agents, respectively, wherein each subtype of bead (each subset) comprises one type of binding agent, recognizing or binding a potential marker on vesicles. The vesicles of said sample which are recognized (and bound) by a capture agent are immobilized, i.e. they are captured.

The captured vesicles are contacted with detection agents, in number between 2 and as many different markers as are of interest. Each detection agent recognizes an potential marker of the vesicles. At least two of the binding agents of the at least two detection agents recognize the same markers as at least two binding agents of the at least two capture agents used in the preceding step. Each used binding agent of the detection agent, e.g. an antibody or fragment thereof, may be labeled with a different fluorophore. Identification of the captured vesicles contacted with the detection agents may be performed e.g. by fluorescent activated cell sorting using devices such as e.g. MACSQuant® Analyzer (Miltenyi Biotec GmbH) resulting in a profile of markers present on the vesicles of said sample. The generated matrix allows to determine levels of the markers of the captured vesicles relative to each other, thereby identifying subpopulations of vesicles if the profile differs between two vesicles. In addition, the profile may be used to determine the proportion of the subpopulations of vesicles in said sample. Capture and detection can be performed subsequently as described above, the detection agent can also be pre-incubated with the vesicles and then incubated with the capture agent or capture and detection can be performed in parallel. To reduce non-specific binding and to improve the signal to noise ratio, it is possible to include washing steps after capture and/or after adding the detection reagent.

In one embodiment of the invention the matrix may be balanced, i.e. a set of at least 2 beads with different capture agents and at least 2 different detection agents recognizing the same markers as the at least 2 capture agents, respectively, may be used. This results in matrices of 2x2, 3x3, 4x4 or higher matrices allowing the detection of co-expressed markers of the vesicles.

In a preferred embodiment, several binding agents will be coupled to a surface to build the capture agent. Therefore, several vesicles can bind to the same surface, i.e. the same single capture agent. The detection agent comprises one binding agent and at least one signaling label. The gained signal will then be influenced by the number of vesicles bound to the surface and the number of marker epitopes per vesicle like depicted in the above examples.

In another embodiment, one could set up the capture agent to allow only a single vesicle to be captured to each surface e.g. by a single binding agent coupled to each surface. Using an excess of these capture agents over the amount of vesicles, the proportion of positive capture agents will be the measure for the proportion of the respective marker within the vesicle population.

For example, a sample comprising two populations of vesicles: A first population of 50% of all vesicles with vesicles carrying 100 entities of marker A and 100 entities of marker B on each vesicle and a second population with the remaining 50% of all vesicles carrying 100 entities of marker A per vesicle but no marker B.

Using capture agent for marker A, 80% of all beads captured vesicles giving a positive signal. But only 40% of beads coupled to capture agent B will give a positive signal as only 50% of all vesicles comprise marker B and can bind to the respective capture agent. Therefore, detection using detection agents specific for marker A with 100 entities on all vesicles will give twice the number of positive capture agents for marker A as compared to the beads coupled to marker B.

If marker B is used for detection, only 50% of the vesicles bound to capture agents specific for marker A will be detected as the remaining vesicles are devoid of marker B and will give no signal. A comparable signal can be expected for capture agent B. In this case, only 50% of the vesicles will be bound, but all these vesicles carry 100 entities marker B and will be detected. In this example, the signal intensity of the positive capture agents will be 100 in any case as 100 entities of marker A and marker b respectively are present on each vesicle.

**Table 9**

| | proportion of positive capture agents by detection agent A | proportion of positive capture agents by detection agent B | Signal intensity detection agent A | Signal intensity detection agent B |
|---|---|---|---|---|
| A bead | 80 | 40 | 100 | 100 |
| B bead | 40 | 40 | 100 | 100 |

Table 9 shows a theoretical example of a 2x2 matrix for marker A and B with marker B being less pronounced per vesicle. The numbers haven been chosen assuming the signal intensity reflecting the numbers of the respective marker per vesicle.

In one embodiment of the invention the matrix may be non-balanced, i.e. a set of at least 2 capture agents, e.g. beads with different binding agents and a number of different detection agents may be used, with the proviso that said number of detection agents is at least two but unequal the number of capture agents used, and wherein at last two detection agents recognize the same markers as the at least two capture agents, respectively. This results in matrices of e.g. 2x3, 2x3, 2x4, 3x2, 4x2, 4x3 or higher matrices allowing the detection of co-expressed markers of the vesicles. Non-balanced matrices will limit the detailed analysis to the markers used for capturing and detection. However, more different subsets of capture agents still allow the detection of these surface marker without requiring additional detection reagents or a larger amount of vesicles. Additional detections agents using markers not present for capture allows detection of markers of interest not included in the multiplex assay giving a higher flexibility.

In another embodiment of the invention, the binding agents of the capture agents are bound to gel particles instead of beads, which can also be analysed by flow cytometry or microscopic imaging.

Another embodiment of the invention uses microarrays with capture agents separated by space and defined by the position within the array (equal to different surfaces). Antibody microarrays to capture and detect surface markers of exosomes have already been described: Jørgensen M, Bæk R, Pedersen S, Søndergaard EKL, Kristensen SR, Varming K. Extracellular Vesicle (EV) Array: microarray capturing of exosomes and other extracellular vesicles for multiplexed phenotyping. Journal of Extracellular Vesicles. 2013;2:10.3402. Detection has been performed using single detection agents or detection agent cocktails. Using our invention, one could use at least two arrays, incubate aliquots of the same vesicle sample and then stain each array with one of at least two detection agents recognizing at least two markers used also for capturing the vesicles on the array. Such a setting would also give a at least 2x2 matrix allowing the analyses of subpopulations and marker distributions.

In another embodiment of the invention the approach could be to use at least two different dyes coupled to the respective binding agent of the detection agent and stain the array with a mixture of labeled detection agents with at least two detection agents recognizing at least two markers used also for capturing the vesicles on the array. In this case, the matrix could be generated from a single array deducing the signal for the respective detection agents according to the differing dye properties.

In another embodiment of the invention fluorescent microscopy is used to analyse different particles as capture agents and collect the staining intensity for each particle. Automated image analysis allows to discriminate particles by size, shape, fluorescent intensity etc. and integrates the signal intensities for each region of interest.

In another embodiment of the invention an ELISA format is used. At least two binding agents of capture agents are bound each to at least two wells of one or more than one multiwell plate(s). Incubating the sample of interest together with the at least two respective detection agents given each capture agent is at least in one well incubated with the respective staining antibody and the other well with the second staining antibody giving an at least 2x2 matrix.

### Definitions

Unless defined otherwise, technical and scientific terms used herein have the same meaning as commonly understood by one of ordinary skill in the art to which this invention belongs.

The term "marker" as used herein refers to a molecule or more complex structures e.g. assemblies of molecules distinguished by binding of a capture and/or detection agent and that is present in or on a vesicle. In case of antibodies or fragments thereof used for capturing and/or detection , the marker would be the respective antigen or epitope.

Preferentially, the marker is present on the membrane of the vesicle, this results in an easier accessibility of the marker by contact with a capture agent and/or detection agent. If the marker is present in the interior of the vesicle then permeabilization of said vesicle is needed prior to contact the marker, i.e. the antigen with the capture and/or detection agent. In biology the term marker often has the meaning of being specific for a cell, thereby defining e.g. a subtype of cells. Contrary thereto, herein the meaning of the term marker does not exclude the possibility that a given marker is present also on another subpopulation of vesicles within a sample. It is sufficient that the marker present e.g. on two different types of vesicles is accessible for the analysis of the relative level of its presence on said vesicles, respectively, by using the method of the present invention.

The term marker entities is used to point out a plurality of markers of the same kind, whereas different markers are simply indicated by the plural form.

The term "vesicle" as used herein refers to all biological structures wrapped with a membrane originated from a membrane of a living cell or from the endosome of a living cell formed by inward budding of the endosome and fusion of these multi vesicular bodies with the cell membrane. This encompass e.g. a cell, preferentially a eukaryotic as a whole, the endomembrane system e.g. endoplasmatic reticulum, the golgi complex, endosomes, lysosomes, secretory vesicles, intracellular compartments like the nucleus, mitochondria, or peroxisomes, or enveloped viruses or extracellular vesicles which are released by cells. The vesicles have markers either in/on the membrane or in the interior of the vesicle which can be exploited for the analysis of subpopulation of vesicles by the method as disclosed herein. Therefore the vesicles analyzed with the present method may be selected from the group consisting of a cell, the endomembrane system of a cell, the intracellular compartments, enveloped viruses and extracellular vesicles.

The term "extracellular vesicles" as used herein refers to diverse types of membrane vesicles of endosomal and plasma membrane origin which are released by cells into the extracellular environment.

The term "enveloped virus" refers to viruses having viral envelopes covering their protective protein capsids. The envelopes typically are derived from portions of the host, but include some viral glycoproteins.

"Capture agent" as used herein comprise a binding agent and a surface to which the binding agent is covalently or non-covalently bound. The binding agent is for binding (recognizing) a marker on a vesicle in a sample comprising a heterogeneous population of vesicles. The surface is for capturing (immobilizing) the vesicle which is recognized by the binding agent of the capture agent.

"Detection agent" (staining agent) as used herein comprise a binding agent and a signaling label. The binding agent is for binding (recognizing) a marker on a vesicle in a sample comprising a heterogeneous population of vesicles. The signaling label is covalently or non-covalently coupled to the binding agent.

"Binding agent" as used herein refers to a moiety or antigen-binding molecule which binds (recognizes) a marker on vesicles in a sample comprising a heterogeneous population of vesicles. A binding agent may be linked to a surface resulting in a capture agent, or a binding agent may be linked to a signaling label resulting in a detection agent.

Binding agents can be described by their binding strength (affinity) and the specificity for a given marker. Affinity is usually defined by the equilibrium dissociation constant or K_{D}-value. The lower the K_{D}-value, the better the affinity of the binding agent. The K_{D}-value is a mean to measures the concentration of binding agent needed to bind the marker (or the marker concentration needed within a sample to be bound by the binding agent). Reasonable antibodies have K_{D} values from micromolar (10⁻⁶ mol/l) to nanomolar (10⁻⁹ mol/l). High affinity antibodies have K_{D} values in the low nanomolar range (10⁻⁹ mol/l) and very high affinity antibodies show picomolar (10⁻¹² mol/l) K_{D} values. Within an assay, the affinities of capture and detection agents will ideally be comparable. It is possible to correct for differing affinities e.g. by determining standard curves.

Binding agents can be e.g. antigen binding molecules. The term "antigen-binding molecule" as used herein refers to any molecule that binds preferably to or is specific for a marker of a vesicle. The term "antigen-binding molecule" comprises e.g. an antibody or antibody fragment. The term "antibody" as used herein refers to polyclonal or monoclonal antibodies, which can be generated by methods well known to the person skilled in the art. The antibody may be of any species, e.g. murine, rat, sheep, human. The antibodies may also be modified antibodies (e.g. oligomers, reduced, oxidized and labeled antibodies).

The term "antibody" comprises both intact molecules and antibody fragments, such as Fab, Fab', F(ab')2, Fv and single-chain antibodies. Additionally, the term "antigen-binding molecule" includes any molecule other than antibodies or antibody fragments that binds preferentially to the marker of a vesicle. Suitable molecules include, without limitation, oligonucleotides known as aptamers that bind to the marker, carbohydrates, lectins or any other antigen binding protein (e.g. receptor-ligand interaction). The linkage (coupling) between antibody or fragment thereof to the surface, e.g. a particle or bead, or to a fluorophore or to enzymatic detectable molecule can be covalent or non-covalent. A covalent (direct) linkage can be, e.g. the linkage to carboxyl-groups on polystyrene beads, or to NH₂ or SH groups on modified beads or other surfaces. A non-covalent (indirect) linkage is e.g. via biotin-avidin or a fluorophore- coupled-particle linked to anti-fluorophore antibody. Methods for coupling antibodies or fragments thereof to surfaces, particles, fluorophores, haptens like biotin are well known to the skilled person in the art.

The terms "specifically binds to" or "specific for" with respect to an antigen-binding molecule, e.g. an antibody or fragment thereof, refer to an antigen-binding molecule (in case of an antibody or fragment thereof to an antigen-binding domain) which recognizes and binds to a specific antigen in a sample, e.g. a marker of vesicles, but does not substantially recognize or bind other antigens, e.g. other markers of vesicles in said sample. An antigen-binding domain of an antibody or fragment thereof that binds specifically to an antigen from one species may bind also to that antigen from another species. This cross-species reactivity is not contrary to the definition of "specific for" as used herein. An antigen-binding domain of an antibody or fragment thereof that specifically binds to an antigen may also bind substantially to different variants of said antigen (allelic variants, splice variants, isoforms etc.). This cross reactivity is not contrary to the definition of that antigen-binding domain as specific for the antigen.

"Signaling label" as used herein refers to molecules which allow the detection of said molecules e.g. by measurable physical or chemical properties.

Different kinds of signaling labels may be magnetic properties, electric conductivity, resistance, light scattering, opaque level or specific signaling labels defined e.g. by colour, colour mixtures, fluorescence, phosphorescence, bioluminescence, chemoluminescence, barcodes, radioactive isotopes, non-radioactive isotopes, nucleic acids, markers, antibodies, enzymatic activity etc. or combinations thereof. The signaling label may be coupled directly or indirectly to the detection agent.

Preferentially, the signaling labels are fluorophores. A fluorophore (or fluorochrome) is a fluorescent chemical compound that can re-emit light upon light excitation. Fluorophores used to label cells or cell components or other biological components are well known in the art. Fluorophores suited for use in the method of the present invention are for e.g., but not limited to fluorescein isothiocyanate (FITC), R-phycoerythrin (PE), Allophycocyanin (APC), PerCP, APC-Vio770, PE-Vio770, VioGreen, VioBlue.

The binding agents can be linked to signaling labels for detection or to discriminate between different detection agents. Using different kinds of signaling labels allows to combine at least two detection agents and use the mixture of at least two detection agents to detect two markers in the same sample. Thereby, capture as well as detection can be performed on the same sample giving a at least 2x2 matrix by one multiplex reaction.

The terms "determining the level of a marker" or "analyzing the level of a marker" as used herein refer to the measurement of the detected amount of marker entities on a vesicle. In most cases, the measurement will be indirect, e.g. a fluoroscent signal due to vesicles bound to the surface and stained by a fluorescently labeled detection agent. In a range suitable for the assay, more vesicles or vesicles comprising more marker molecules will give an increased fluorescent signal. In most cases the level of a marker on a vesicle is determined and/or analyzed relative to another marker on the same vesicle. Depending on the affinity of capture and detection agent and on the used fluorescent dye, signal intensities can differ for two markers, even if the number or concentration of the two markers in the sample is the same. The term "subpopulation of the vesicles" as used herein means a subset of vesicles within a sample comprising vesicles differing from another subset of the vesicles of said sample by the absence or a distinguished level of presence of at least one marker used within the method of the present invention.

The term "heterogeneous population of vesicles" as used herein refers to at least two subpopulations of vesicles within a sample.

The term "sample comprising a heterogeneous population of vesicles" as used herein refers to any sample which comprises at least two types of vesicles in any ratio, e.g. two types of exosomes. In addition, the sample may comprise other components than vesicles. The sample may be originated from animals, especially mammals such as mouse, rats or humans. The sample may be a biological sample , e.g. blood, cerebrospinal fluid, mucous, bone marrow aspirate purulent discharge, biopsy or surgical excision of solid tissue, tissue culture medium or ascite fluid. The vesicles, e.g. exosomes, may be isolated (pre-enriched) from the biological sample by methods well-known in the art before they are contacted with the capture agents. The generated pre-enriched sample is also a sample comprising a heterogeneous population of vesicles".

The term "signal intensities" as used herein refers to the intensities of signals which can be measured from the signal labels of the detection agents. Normally, the measurement is done by a device suitable to measure (detect) the signals of the signal labels, e.g. the fluorometric measurement of fluorophores by a fluorometer in a flow sorter such as MACSQuant Analyzer. Regularly, the difference in intensities of signals measured reflects the amount of bound detection agents to the respective marker of the vesicles.

The term "negative signal intensities" describes signal intensities below a given threshold. To define threshold values, negative control samples comprising buffer instead of a sample comprising vesicles or capture and/or detection agents recognizing markers not present on the sample comprising vesicles.

The term "surface", as used herein refers to any surface of having a capture agent attached thereto and includes, for example, metals glass, plastics, copolymers, colloids, Essentially any surface that is capable of retaining a capture agent bound or attached thereto. The surface may be a particle or bead, e.g. a magnetic bead. The surface may also be the surface of a microarray having multiple positions of different capture agents on it. Then, each position of the respective capture agent represents a subset of the capture agents.

The term "swapping" describes the fact that at least two binding agents are used as capture agent and detection agent respectively. Thereby at least four combinations of capture and detection agents with at least one pair having the same binding agent for capture and detection and at least one pair has different binding agents used for capture and detection. For the mixed pair, the one binding agent (A) of the one capture agent is used as detection agent (A) for the other capture agent (B) and the other binding agent (B) of the other capture agent (B) if used as detection agent (B) for the one capture agent (A) giving AB and the swapped combination BA.

Depending on the number of interesting markers, more than two pairs of capture and detection agent can be swapped.

A "cocktail of antibodies" as used herein means a mixture of antibodies specific for different markers.

The term "matrix" as used herein refers to a rectangular array of numbers arranged in rows (horizontal lines) and columns (vertical lines). Regularly, the individual items in a matrix are called elements or entries. As used herein the elements of the matrix are the measured signal intensities of the respective signal labels of the detection agents used within the method of the present invention. One may arrange the signals of a respective detection agent in a column and correlate the signals to the respective capture agents given in rows a vice versa. The following is an example of a matrix with 2 rows and 3 columns with six elements in sum (a "2x3 matrix"):

**Table 10**

| | Detection agent 1 | Detection agent 2 | Detection agent 3 |
|---|---|---|---|
| Capture agent 1 | 24.7 LU | 13.3 LU | 6.1 LU |
| Capture agent 2 | 23.3 LU | 18.1 LU | 1.1 LU |

Table 10 shows a theoretical example of a 2x3 matrix for marker 1, 2 and 3.

The numbers given in the exemplary matrix are arbitrary light units (LU). The presentation of the elements of a matrix can be in any manner which is suited to analyze the signal intensities generated by the method of the present invention.

A "balanced matrix" as used herein refers to a matrix consisting of data generated by using pairs of capture agents and detection agents which are swapped, respectively giving a matrix with the same number of rows and columns e.g. 2x2, 3x3, 4x4 etc..

A "non-balanced matrix" or a "not balanced matrix" as used herein refers to a matrix consisting of data generated by using at least one pair of capture agent and detection agent which are swapt, respectively and at least one additional capture or detection agent with the binding agent used solely for capture or detection. Thereby, a matrix with the number of rows being not equal to the number of columns e.g. 2x3, 3x2, 2x5, 5x2 etc..

A symmetric matrix as used herein refers to a matrix with the signal intensities are not affected by swapping the pair of binding agents. Using capture agent for marker A and detection agent for marker B will give the same signal as capture agent for marker B and detection agent for marker A.

An asymmetric matrix as used herein refers to a matrix with the signal intensities are affected by swapping the pair of binding agents. Using capture agent for marker A and detection agent for marker B will give altered signals as compared to capture agent for marker B used in combination with detection agent for marker A.

### Examples

### Example 1: Protein profile of platelet vesicles

Whole blood was diluted with the equal volume of Krebs Ringer buffer (100 mM NaCl, 4 mM KCI, 20 mM NaHCO₃, 2 mM Na₂SO₄, 4.7 mM citric acid, 14.2 mM tri-sodium citrate) at pH 7.4 and centrifuged at 190xg for 10 min. The platelet-rich plasma was centrifuged at 100xg for 20 min and platelets were pelleted from the supernatant at 1,000xg for 15 min and washed twice with Krebs Ringer buffer. 1x10⁹ platelets per mL were activated with 50 nM Calcium Ionophore and 10 mM calcium chloride for 30 min at room temperature (Leong et al. 2011, Validation of flow cytometric detection of platelet microparticles and liposomes by atomic force microscopy, J of Thrombosis and Haemostasis, 9: 2466-2476). The platelet suspension was centrifuged at 2,000xg for 30 min and at 10,000xg for 45 min and filtrated through a 0.22 µm sterile filter. Protein concentration was determined by BCA Assay (Thermo Fisher Scientific, MA, USA) and BSA as standard.

A set of 39 differently labeled beads (Miltenyi Biotec, Germany) coupled to capture antibodies were incubated over night with 16 µg protein of platelet supernatant in a MACSmix Tube Rotator (Miltenyi Biotec, Germany). After washing with MACSQuant Running Buffer (Miltenyi Biotec, Germany) the beads were pelleted by centrifugation at 3000xg for 5 min and resuspended in 100 µL MACSQuant Running Buffer (Miltenyi Biotec, Germany). Bound vesicles were stained with a cocktail of antiCD9-APC (Miltenyi Biotec, Germany), antiCD63-APC (Miltenyi Biotec, Germany) and antiCD81-APC (BD) or with each detection antibody alone. Buffer was incubated with the beads and stained as background control. The flow cytometer MACSQuant Analyzer 10 with the corresponding software (Miltenyi Biotec, Germany) was used for analysis. The signals of the buffer control were subtracted from the sample signals per bead type to assess background corrected signals.

The median signals for antiCD81-beads were relatively low in comparison to the antiCD63-and antiCD9-beads independent of the staining antibody. The median signals of the antiCD81-APC staining of all bead types were also very low indicating no or at least a far lower level of CD81 on platelet vesicles. The successful detection of vesicles with other combinations of beads and detection antibodies proves the functionality of the antibodies. Furthermore, the staining with different detection antibodies allow an estimation of the frequency of markers on the surface of platelet vesicles. For example the median signals after the capture with antiCD63-beads and detection with antiCD9-APC were stronger in comparison to the combination of antiCD9-beads with antiCD63-APC. This implies that the vesicles harbor enough CD63 to be captured by antiCD63-beads and many CD9 to give a strong signals after antiCD9-staining. But the signals were lower if the vesicles were captured by their numerous CD9 with antiCD9-beads and the less prevalent CD63 epitopes were stained with antiCD63-APC, meaning that CD9 was more frequent on platelet vesicles than CD63.

In conclusion, platelet vesicles seem to harbor an asymmetric protein profile with low CD81 and lower CD63 than CD9 occupancy (see FIG 1).

### Example 2: Asymmetric protein profile of Natural Killer (NK) cell exosomes

NK cells were isolated from buffy coats by removing non-target cells by immunomagnetic depletion with the MACSxpress NK cell Isolation Kit (Miltenyi Biotec, Germany). NK cells were cultivated in TexMACS medium (Miltenyi Biotec, Germany) with 5% human AB serum and 500 IU/mL Proleukin and EBV-LCLs as feeder cells. Medium was renewed on day 7 and 9 when feeder cells were no longer detectable. Supernatants were harvested on day 12, centrifuged at 2,000xg for 30 min and at 10,000xg for 45 min and filtrated through a 0.22 µm sterile filter. Exosomes were pelleted by ultracentrifugation for 2 h at 100,000xg, washed with PBS and then resuspended in 1/500 of the initial volume of PBS. Protein concentration was determined by BCA Assay (Thermo Fisher Scientific, MA, USA) and BSA as standard.

A set of 39 differently labeled beads (Miltenyi Biotec, Germany) coupled to capture antibodies were incubated over night with 16 µg protein ofNK cell exosomes in a MACSmix Tube Rotator (Miltenyi Biotec, Germany). The beads were washed with MACSQuant Running Buffer (Miltenyi Biotec, Germany) and pelleted by centrifugation at 3000 xg for 5 min before resuspension in 100 µL MACSQuant Running Buffer (Miltenyi Biotec, Germany). Bound vesicles were stained with a cocktail of antiCD9-APC (Miltenyi Biotec, Germany), antiCD63-APC (Miltenyi Biotec, Germany) and antiCD81-APC (BD) or with each detection antibody alone. Buffer was incubated with the beads and stained as background control. The flow cytometer MACSQuant Analyzer 10 with the corresponding software (Miltenyi Biotec, Germany) was used for analysis.

The signals of the buffer control were subtracted from the sample signals per bead type to assess background corrected signals.

The median signals for antiCD9-beads were relatively low in comparison to the antiCD63-and antiCD81-beads independent of the staining antibody. The median signals of the antiCD9-APC staining of all bead types were also low indicating a lower level of CD9 on NK cell vesicles. The successful detection with other combinations of beads and detection antibodies proves the functionality of the antibodies.

Furthermore, the staining with different detection antibodies allows presumptions about the frequency of epitopes on the surface of NK cell vesicles. For example the median signals after the capture with antiCD63-beads and detection with antiCD81-APC were stronger in comparison to the combination of antiCD81-beads with antiCD63-APC. In addition, the signals of antiCD63-beads were slightly higher than those of antiCD81-beads after staining with the antiCD9/antiCD63/antiCD81-cocktail. This implies that NK cell exosomes display a low amount of CD9 markers and a lower amount of CD81 than CD63 (see FIG 2).

### Example 3: Identification of subpopulations in a mixture of exosomes

To simulate a mix of different subpopulations of exosomes, a 1:1 mixture was created with NK cell exosomes and platelet vesicles generated as described in example 1 and 2.

A set of 39 differently labeled beads (Miltenyi Biotec, Germany) coupled to capture antibodies were incubated over night with a mixture of 8 µg protein of NK cell exosomes and 8 µg protein of platelet supernatant in a MACSmix Tube Rotator (Miltenyi Biotec, Germany). The beads were washed with MACSQuant Running Buffer (Miltenyi Biotec, Germany) and pelleted by centrifugation at 3000 xg for 5 min before resuspension in 100 µL MACSQuant Running Buffer (Miltenyi Biotec, Germany). Bound vesicles were stained with a cocktail of antiCD9-APC (Miltenyi Biotec, Germany), antiCD63-APC (Miltenyi Biotec, Germany) and antiCD81-APC (BD) or with each detection antibody alone. Buffer was incubated with the beads and stained as background control. The flow cytometer MACSQuant Analyzer 10 with the corresponding software (Miltenyi Biotec, Germany) was used for analysis.

The signals of the buffer control were subtracted from the sample signals per bead type to assess background corrected signals.

The protein profiles of platelet vesicles and NK cell exosomes as described in example 1 and 2 could be recovered in this 1:1 mixture: the median signals for antiCD9-beads stained with antiCD81-APC and of antiCD81-beads stained with antiCD9-APC were the lowest reflecting that platelet vesicles and NK cell exosomes were either CD81-positive or CD9-positive, respectively, but not double positive. Therefore, the two subpopulations in this simulated mixture could be discriminated by staining with the different exosome markers separately (see

FIG 3).

### Example 4: Protein profile of monocyte exosomes

Monocytes were isolated from PBMCs by immunomagnetic cell sorting using CD14-MicroBeads (Miltenyi Biotec, Germany). Monocytes were cultivated in RPMI1640 (Miltenyi Biotec, Germany) with 5% exosome-depleted FCS and 2mM L-Glutamine for 24 h. Supernatants were centrifuged at 2,000xg for 30 min and at 10,000xg for 45 min and filtrated through a 0.22 µm sterile filter. Exosomes were pelleted by ultracentrifugation for 2 h at 100,000xg, washed with PBS and then resuspended in 1/400 of the initial volume of PBS. Protein concentration was determined by BCA Assay (Thermo Fisher Scientific, MA, USA) and BSA as standard.

A set of 39 differently labeled beads (Miltenyi Biotec, Germany) coupled to capture antibodies were incubated over night with 8 µg protein of monocyte exosomes in a MACSmix Tube Rotator (Miltenyi Biotec, Germany). The beads were washed with MACSQuant Running Buffer (Miltenyi Biotec, Germany) and pelleted by centrifugation at 3000 xg for 5 min before resuspension in 100 µL MACSQuant Running Buffer (Miltenyi Biotec, Germany). Bound vesicles were stained with a cocktail of antiCD9-APC (Miltenyi Biotec, Germany), antiCD63-APC (Miltenyi Biotec, Germany) and antiCD81-APC (BD) or with each detection antibody alone. Buffer was incubated with the beads and stained as background control. The flow cytometer MACSQuant Analyzer 10 with the corresponding software (Miltenyi Biotec, Germany) was used for analysis.

The signals of the buffer control were subtracted from the sample signals per bead type to assess background corrected signals.

In contrast to the analyzed NK cell exosomes and platelet exosomes in Examples 1 and 2, monocyte exosomes showed signals on CD9-, CD63- as well as on CD81-beads. But differences in signals still indicate an unequal distribution of the so called exosome markers CD9, CD63 and CD81. For example, after staining with a cocktail of CD9/CD63/CD81-APC antibodies the highest signals were obtained with CD63-beads pointing to CD63 as the most frequently exosome marker on monocyte exosomes. CD63 is followed by CD9 in incidence, demonstrated by high signal intensities after staining with CD9-APC antibody of any capture bead except CD9-beads likely caused by blocking of the same recognized epitope. In each dimension, lower signals were obtained with CD81-beads or CD81-APC staining, respectively, indicating CD81 as third frequent exosome marker on monocyte exosomes.

### Example 5: Analysis ofNK cells

To demonstrate that the invention is applicable to biological spheres of different sizes, large NK cells (approximately 10 µm) were analyzed in addition to relatively small exosomes (60-100 nm).

A set of 39 differently labeled beads (Miltenyi Biotec, Germany) coupled to capture antibodies were incubated over night with 5x10⁶ NK cells in a MACSmix Tube Rotator (Miltenyi Biotec, Germany). The beads were washed with MACSQuant Running Buffer (Miltenyi Biotec, Germany) and pelleted by centrifugation at 3000 xg for 5 min before resuspension in 100 µL MACSQuant Running Buffer (Miltenyi Biotec, Germany). Bound vesicles were stained with antiCD56-APC (Miltenyi Biotec, Germany). Buffer was incubated with the beads and stained as background control. The flow cytometer MACSQuant Analyzer 10 with the corresponding software (Miltenyi Biotec, Germany) was used for analysis.

The signals of the buffer control were subtracted from the sample signals per bead type to assess background corrected signals.

The strongest signal was measured on beads with the NK cell marker antiCD56 as capture antibody as expected. NK cells could also be detected on some other capture antibody beads proving the applicability of the MACSPlex Exosome Kit not only for small vesicles of exosome size but also for large cells like NK cells.

Similar results can be gained using different detection agents again allowing more detailed analysis of the marker composition as shown in the exosome examples above.

## Claims

1. A method for analysing levels of markers of subpopulations of vesicles in a sample comprising a heterogeneous population of said vesicles, the method comprising
a) Contacting said sample with a set of at least two capture agents, wherein each distinguishable subset of said set of capture agents comprises a different binding agent or combination of binding agents, and wherein each different binding agent recognizes a different marker of said vesicles; thereby capturing at least two subpopulations of said vesicles of said sample
b) Contacting the captured at least two subpopulations of vesicles with at least two different detection agents, wherein each different detection agent comprises a different binding agent, and wherein each different binding agent recognizes a different marker of said vesicles, and wherein at least two of said different binding agents of said at least two detection agents recognize the same at least two markers of said vesicles as two of said different binding agents of said at least two capture agents, and wherein each binding agent of said detection agents is labeled with different or the same signaling labels; thereby labeling at least two markers of said at least two subpopulations of said vesicles inversely with said at least two capture agents and at least two detection agents, thereby generating an at least 2x2 matrix of signal intensities
c) Determining the levels of said at least two markers of said at least two subpopulations of said vesicles, wherein said levels of said at least two markers of subpopulations of said vesicles are determined relative to each other.

2. The method according to claim 1, wherein said signaling labels are fluorophores, quantum dots, radioactive signals or enzymes generating detectable signals..

3. The method according to claim 1 or 2, wherein said determining the levels of the markers may be performed by using a device capable of measurement of fluorescence intensities.

4. The method according to claim 3, wherein the highest signal obtained by said measurement of one or a combination of the detection agent(s) is set to 100% and all other relative signals intensities are calculated accordingly therefrom.

5. The method according to any one of claims 1 to 4, wherein said vesicles are cells or extracellular vesicles, intracellular compartments of a cell or enveloped viruses.

6. The method according to any one of claims 1 to 5, wherein said set of capture agents is a set of bead.

7. The method according to claim 6, wherein said distinguishable subset of said set of beads may be differently stained with dyes and /or may be different in size.

8. The method according to claim 6 or 7, wherein in said capture agents the binding agents are covalently coupled to said beads.

9. The method according to any one of claims 1 to 8, wherein said binding agents are antibodies or fragments thereof.

10. The method according to any one of claims 1 to 9, wherein said generated matrix is a balanced matrix.

11. The method according to any one of claims 1 to 9, wherein said generated matrix is a non-balanced matrix.
